# EUROPÄISCHE PATENTSCHRIFT

(11) **EP 3 198 264 B1**
(45) Veröffentlichungstag und Bekanntmachung des Hinweises auf die Patenterteilung: **07.10.2020**
(21) Anmeldenummer: 15753043.7
(22) Anmeldetag: 20.08.2015
(51) Int. Cl.: G01N 24/08, G01R 33/32, G01R 33/30, A61B 5/08, G01N 33/22, G01N 33/497, B01L 3/00, G01R 33/24, G01N 24/10

(54) **ATEMGASANALYSEGERÄT ZUR NMR-ANALYSE VON SUBSTANZEN IN EINER PROBE UND VERFAHREN ZUR ATEMGASANALYSE**
RESPIRATORY GAS ANALYSER FOR THE NMR ANALYSIS OF SUBSTANCES IN A SAMPLE, AND METHOD FOR RESPIRATORY GAS ANALYSIS
APPAREIL D'ANALYSE RMN DE GAZ RESPIRATOIRE, ET PROCÉDÉ D'ANALYSE DES GAZ RESPIRATOIRES

(30) Priorität: 26.09.2014 DE 102014219561
(43) Veröffentlichungstag der Anmeldung: 02.08.2017
(73) Patentinhaber: Robert Bosch GmbH, 70442 Stuttgart (DE)
(72) Erfinder: LUTZ, Theresa, 68163 Mannheim (DE); EINSELE, Florian, 70180 Stuttgart (DE); LUCKERT, Katrin, 71229 Leonberg (DE); ROELVER, Robert, 75365 Calw-Stammheim (DE)
(86) Internationale Anmeldenummer: PCT/EP2015/069143
(87) Internationale Veröffentlichungsnummer: WO 2016/045888

(56) Entgegenhaltungen:
- GB-A- 2 483 767
- US-A1- 2009 275 852
- US-A1- 2011 062 957
- US-A1- 2011 120 890
- MAMIN H J ET AL: "Nanoscale nuclear magnetic resonance with a nitrogen-vacancy spin sensor", SCIENCE AMERICAN ASSOCIATION FOR THE ADVANCEMENT OF SCIENCE USA, Bd. 339, Nr. 6119, 1. Februar 2013 (2013-02-01), Seiten 557-560, XP002747960, ISSN: 0036-8075
- ACOSTA VICTOR M ET AL: "Broadband magnetometry by infrared-absorption detection of diamond NV centers and associated temperature dependence", ADVANCES IN PHOTONICS OF QUANTUM COMPUTING, MEMORY, AND COMMUNICATION IV, SPIE, 1000 20TH ST. BELLINGHAM WA 98225-6705 USA, Bd. 7948, Nr. 1, 10. Februar 2011 (2011-02-10), Seiten 1-9, XP060007631, DOI: 10.1117/12.872624 [gefunden am 2011-02-11]
- HAW J F ET AL: "CONTINUOUS FLOW HIGH FIELD NUCLEAR MAGNETIC RESONANCE DETECTOR FOR LIQUID CHROMATOGRAPHIC ANALYSIS OF FUEL SAMPLES", ANALYTICAL CHEMISTRY, AMERICAN CHEMICAL SOCIETY, US, Bd. 53, Nr. 14, 1. Dezember 1991 (1991-12-01), Seiten 2327-2332, XP000940993, ISSN: 0003-2700, DOI: 10.1021/AC00237A045

## Beschreibung

Die vorliegende Erfindung betrifft eine Einrichtung zur Analyse von Substanzen in einer Probe, wobei die Analyse auf einer Messung von magnetischen Kernspinresonanzen beruht, sowie ein Atemgasanalysegerät, einen Kraftstoffsensor und ein Verfahren zur Analyse von Substanzen in einer Probe.

### Stand der Technik

Die Kernspinresonanzspektroskopie (NMR-Spektroskopie, *Nuclear Magnetic Resonance*) ist eine vielfach angewendete Methode zur Untersuchung der elektronischen Umgebung einzelner Atome und der Wechselwirkungen von Atomen untereinander. Die Methode beruht dabei auf der sogenannten magnetischen Kernspinresonanz. Hiermit wird eine resonante Wechselwirkung zwischen dem magnetischen Moment von Atomkernen, die sich in einem starken statischen Magnetfeld befinden, mit einem hochfrequenten magnetischen Wechselfeld beschrieben. Hierbei absorbieren und emittieren die Atomkerne einer Materialprobe in einem konstanten Magnetfeld elektromagnetische Wechselfelder. Anhand von charakteristischen Frequenzverschiebungen der durch das magnetische Moment der Kernspins hervorgerufenen Spin-Präzision kann beispielsweise auf den Bindungszustand von bestimmten Isotopen in organischen Molekülen geschlossen werden. Die Kernspinresonanzspektroskopie ist prinzipiell in der Lage, beliebige organische Verbindungen nachzuweisen. Es sind aber nur solche Isotope der Spektroskopie zugänglich, die im Grundzustand einen von 0 verschiedenen Kernspin und damit ein magnetisches Moment besitzen. Hierzu zählen beispielsweise ¹H und ¹³C. Die magnetischen Momente präzidieren beim Anlegen eines statischen Magnetfeldes mit einer für das jeweilige Atom charakteristischen Frequenz, die sich im Allgemeinen in einem Bereich zwischen kHz und MHz bewegt. Sie senden also ein magnetisches Wechselfeld im Bereich von wenigen pT aus. Da die Frequenz des Wechselfeldes neben der Atomsorte auch vom Bindungszustand des Atoms abhängig ist, führen die Bindungszustände des Atoms zu Frequenzverschiebungen im Bereich von wenigen Hz bis 1000 Hz. Auf Grund der begrenzten Sensitivität von Magnetfeldsensoren ist derzeit eine praktische Umsetzung nur mit sehr großen Magnetfeldern möglich, um ausreichend viele Spins zu polarisieren, so dass bei entsprechender Pulsanregung ein ausreichend hohes Messsignal erhalten werden kann. Die erforderlichen Magnetfelder, die bis zu 10 T erreichen müssen, können beispielsweise mit supraleitenden Magneten, die mit Flüssigstickstoff gekühlt werden, bereitgestellt werden. Aus diesem Grund sind Miniaturisierungen von NMR-Geräten in der Regel nicht möglich.

Die internationale Patentanmeldung WO 2012/016977 A2 befasst sich mit einem Verfahren zur Herstellung eines optischen Elements auf der Basis von Diamant.

Als mögliche Anwendung eines solchen optischen Elementes wird ein Magnetometer beschrieben.

Aus GB2483767 ist eine mikrofluidische Einrichtung zur Analyse einer fluidischen Probe mittels Stickstoff-Vakanz-Zentren in Diamant bekannt. Aus MAMIN H J ET AL, "Nanoscale nuclear magnetic resonance with a nitrogen-vacancy spin sensor", SCIENCE AMERICAN ASSOCIATION FOR THE ADVANCEMENT OF SCIENCE USA, (20130201), vol. 339, no. 6119, ISSN 0036-8075, pages 557 - 560, XP002747960 [X] 1,2,10-12,15 ist ebenfalls ein Stickstoff-Vakanz-Zentren basierender Sensor bekannt. Ferner offenbaren auch US2011120890 A1 und ACOSTA VICTOR M ET AL, "Broadband magnetometry by infrared-absorption detection of diamond NV centers and associated temperature dependence", ADVANCES IN PHOTONICS OF QUANTUM COMPUTING, MEMORY, AND COMMUNICATION IV, SPIE, 1000 20TH ST. BELLINGHAM WA 98225-6705 USA, (20110210), vol. 7948, no. 1, doi:10.1117/12.872624, pages 1 - 9, XP060007631 ein solchen Sensor. Ein Gerät zur Atemgasanalyse, insbesondere mittels NMR, ist beispielsweise aus US2009/0275852 A1 bekannt.

### Offenbarung der Erfindung

### Vorteile der Erfindung

Die Erfindung stellt ein Atemgasanalysegerät mit einer Analyseeinrichtung zur Analyse von Substanzen in einer Atemgasprobe auf der Basis einer Messung von magnetischen Kernspinresonanzen bereit, die für eine Vielzahl von Anwendungen einsetzbar ist und vor allem auch einer Miniaturisierung zugänglich ist. Die erfindungsgemäße Einrichtung, welche in Anspruch 1 definiert ist, weist zunächst Mittel zur Erzeugung eines Referenzmagnetfeldes auf, mit dem Magnetresonanzen in den Atomen der Substanzen in der Probe induziert werden. Weiterhin weist die erfindungsgemäße Einrichtung wenigstens einen Magnetfeldsensor auf, der dadurch gekennzeichnet ist, dass er wenigstens eine sensitive Komponente mit Diamantstrukturen umfasst, wobei die Diamantstrukturen Stickstoff-Vakanz-Zentren (NV-Zentren) aufweisen. Die Stickstoff-Vakanz-Zentren in der Diamantstruktur zeichnen sich durch eine auslesbare elektronische Struktur aus, die durch die Wirkung von Magnetfeldern veränderbar sind. Diese Veränderungen können gemessen werden und stellen die Basis für die Analyse der Substanzen in der Probe dar. Die Erfindung nutzt damit die Eigenschaften von NV-Zentren in Diamant zur Messung von Kernspin-Resonanzen. Durch die Verwendung von Diamantstrukturen mit NV-Zentren kann ein sehr sensitiver Magnetfeldsensor bereitgestellt werden, der in der Lage ist, die magnetischen Wechselfelder im Bereich von wenigen pT, die als Kernspin-Resonanzen auftreten können, zu messen. Aus einer wissenschaftlichen Veröffentlichung von Jelezko et al. (Phys. Stat. Sol. (a) 203, No. 13, 3207-3225 (2006)) ist bereits bekannt, dass Stickstoff-Vakanz-Zentren (NV-Zentren) in Diamant eine charakteristische elektronische Struktur aufweisen, die durch eine Bestrahlung mit Mikrowellenstrahlen und eine Bestrahlung im optischen Bereich angeregt und durch Detektion einer von den NV-Zentren emittierten Fluoreszenz wieder ausgelesen werden kann. Die elektronische Struktur ist von der Gitterkonstante des Diamantkristalls und auch von Magnetfeldeinwirkungen abhängig. Die Gitterkonstante wird von der Temperatur und von Kristallverspannungen beeinflusst, wobei die Kristallverspannungen beispielsweise durch Druckeinwirkungen verändert werden können. Die Erfinder konnten zeigen, dass die elektronische Struktur der NV-Zentren unter anderem sehr empfindlich auf äußere Magnetfelder reagiert. Insbesondere lassen sich bei entsprechender Auslesung der elektronischen Struktur Magnetfelder mit einer Sensitivität von bis zu 100 pT/√Hz messen. Dieses sehr empfindliche Messprinzip eignet sich damit in besondere Weise für die Detektion von Kernspinresonanzen, wobei mit wesentlich schwächeren Magnetfeldern als bisher üblich gearbeitet werden kann.

Der Magnetfeldsensor der erfindungsgemäßen Einrichtung weist wenigstens ein Mittel zur Einkopplung von elektromagnetischer Anregungsstrahlung im optischen Bereich, insbesondere in einem Wellenlängenbereich zwischen etwa 530 nm und etwa 570 nm (grüner Bereich des sichtbaren Lichts), und wenigstens ein Mittel zur Einkopplung von elektromagnetischer Strahlung im Mikrowellenbereich, insbesondere in einem Frequenzbereich zwischen etwa 2000 MHz und etwa 4000 MHz, auf. Durch eine Anregung mit elektromagnetischer Strahlung im optischen Bereich und einer elektromagnetischen Anregung im Mikrowellenbereich wird in den NV-Zentren der Diamantstrukturen eine Fluoreszenzemission induziert, deren Spektrum von dem wirkenden Magnetfeld, insbesondere von der Magnetfeldstärke, abhängt. Zur Erfassung der emittierten Fluoreszenzstrahlung weist der Magnetfeldsensor der erfindungsgemäßen Einrichtung weiterhin wenigstens ein Mittel zur Detektion der emittieren Fluoreszenzstrahlung auf. Um die Auswertung zu erleichtern, ist weiterhin vorzugsweise wenigstens ein Mittel zur Filterung von elektromagnetischer Strahlung vorgesehen, wobei es sich hierbei vorzugsweise um eine in den Magnetfeldsensor integrierte optische Filterschicht handelt, der zur Filterung der Anregungsstrahlung aus der emittierten Fluoreszenzstrahlung vorgesehen ist.

Als Mittel für die Einkopplung von elektromagnetischer Strahlung im optischen Bereich kann beispielsweise eine LED (*light-emitting diode*) und/oder ein VCSEL (*vertical-cavity surface-emitting laser*), also ein Halbleiterlaser als Oberflächenemitter, eingesetzt werden. Als Mittel zur Einkopplung von elektromagnetischer Strahlung im Mikrowellenbereich kann beispielsweise eine Streifenantenne vorgesehen sein. Die Streifenantenne kann beispielsweise direkt auf der sensitiven Komponente oder in unmittelbarer räumlicher Nähe dazu angeordnet sein. Zur Detektion der emittieren Fluoreszenzstrahlung ist vorzugsweise eine Photodiode vorgesehen, beispielsweise eine in den Sensor integrierbare p-n-Photodiode, die sich in besonderer Weise für miniaturisierte Anwendungen eignet.

Bei der sensitiven Komponente handelt es sich insbesondere um eine Diamantschicht mit NV-Zentren oder um eine entsprechend beschichtete Membran. Die NV-Zentren der Diamantstrukturen können beispielsweise durch eine NV-Dotierung von Diamant bereitgestellt werden.

In einer besonders bevorzugten Ausgestaltung der erfindungsgemäßen Einrichtung umfasst der Magnetfeldsensor eine Anordnung von mehreren individuell auszuwertenden sensitiven Komponenten. Dem liegt zugrunde, dass das Detektionsvolumen, in welchem sich ein Kernspin um ein NV-Zentrum befinden muss, sehr klein ist, beispielsweise im Bereich um 25 nm³. Durch die Zusammenfassung von mehreren individuell auszuwertenden sensitiven Komponenten, gewissermaßen von mehreren NV-Sensoren, kann die Messbarkeit daher verbessert werden. Die Größe der Anordnung (*Array*) kann vorzugsweise an die Anforderungen angepasst werden, insbesondere an die erforderliche Konzentrationssensitivität für eine bestimmte nachzuweisende Substanz bzw. für eine bestimmte Molekülspezies.

In bevorzugter Weise kann die sensitive Komponente, also die Diamantstrukturen (Diamantflächen) der sensitiven Komponente, Strukturen für eine Oberflächenvergrößerung aufweisen, beispielsweise nadelartige oder röhrenartige Strukturen. Hierdurch kann das Detektionsvolumen pro NV-Zentrum erhöht werden, da durch eine Strukturierung der Diamantflächen Strukturen bereitgestellt werden, die von der zu messenden Probe umschlossen werden können. Geeignete Oberflächen können beispielsweise durch ein reaktives lonenätzen bereitgestellt werden, durch das insbesondere säulenförmig strukturierte Diamantspitzen hergestellt werden können.

In einer weiteren bevorzugten Ausgestaltung der erfindungsgemäßen Einrichtung weist die sensitive Komponente Kapillarstrukturen auf. Für eine Frequenzauflösung von wenigen Hz ist im Allgemeinen eine verhältnismäßig lange Messdauer erforderlich. Durch die Bereitstellung von Kapillarstrukturen wird dabei erreicht, dass sich die zu messende Probe ausreichend lange, d.h. im Zeitraum einiger Sekunden, im Detektionsvolumen des NV-Zentrums aufhält. Durch eine Strukturierung von Kapillaren oder Ähnlichem in der Diamantoberfläche kann damit die Flüssigkeitsprobe im Bereich der NV-Zentren festgehalten werden.

In einer besonders zweckmäßigen Ausgestaltung kann der sensitiven Komponenten wenigstens ein Element zum Heizen und/oder Kühlen, beispielsweise ein heizbares Peltier-Element, zugeordnet sein. Durch ein Beheizen und insbesondere durch eine integrierte Heizung kann beispielsweise die Probenflüssigkeit von der sensitiven Komponente entfernt, d.h. verdampft, werden. Wenn beispielsweise Kapillarstrukturen zum Festhalten der flüssigen Probe vorgesehen sind, kann durch ein Beheizen der sensitiven Komponente nach Beendigung der Messung die Probe verdampft werden. Eine Integration von Heizelementen in die erfindungsgemäße Einrichtung kann darüber hinaus für eine Regenerierung des Magnetfeldsensors genutzt werden, indem beispielsweise anhaftende Partikel thermisch zerstört und entfernt werden. Auf diese Weise kann ein Reset des Sensors durchgeführt werden.

Eine Kühlung der sensitiven Komponente kann vorteilhaft sein, um insbesondere nach einem Beheizen der sensitiven Komponente wieder eine definierte Temperatur bereitzustellen, so dass definierte Messbedingungen eingestellt werden. Eine Kühlung der sensitiven Komponente kann insbesondere auch für solche Anwendungen vorteilhaft sein, bei denen eine Kondensierung von gasförmigen Proben vorgesehen ist, bevor die Probe erfindungsgemäß analysiert wird. Durch Kühlelemente kann dabei eine sogenannte Kühlfalle realisiert werden.

In einer weiteren bevorzugten Ausgestaltung der erfindungsgemäßen Einrichtung umfasst die Einrichtung Mittel zur Ionisation der Probe. Dem liegt zugrunde, dass die Diamantoberfläche, also die Oberfläche der sensitiven Komponente, in der Regel eine negative Oberflächenladung besitzt. Durch eine Ionisation der Probe kann eine Oberflächenadsorption an der geladenen Diamantoberfläche begünstigt werden. Die Ionisation der Probe kann beispielsweise durch starke elektrostatische Felder im Bereich des Probeneinlasses der Einrichtung erreicht werden. Eine Ionisation der Probe ist insbesondere bei gasförmigen Proben vorteilhaft.

Weiterhin kann es mit Vorteil vorgesehen sein, dass die Einrichtung Mittel zur Erzeugung eines positiven Potenzials an der sensitiven Komponente umfasst. Beispielsweise durch Anbringen einer Feldplatte auf der Rückseite der Diamantoberfläche kann die sensitive Komponente, also die Diamantschicht, zwischenzeitlich auf ein hohes positives Potenzial gebracht werden, wodurch nach abgeschlossener Messung die Desorption der Substanzen aus der Probe und ein Sensorreset erreicht werden kann. Um den optischen Zugang durch die sensitive Komponente hindurch zu gewährleisten, kann diese Feldplatte beispielsweise aus transparentem leitfähigem Oxid bestehen, beispielsweise aus Indiumzinnoxid oder Aluminium-dotierten Zinkoxid.

Die erfindungsgemäße Einrichtung eignet sich aufgrund ihrer hohen Messsensitivität und aufgrund der Bandbreite der nachzuweisenden Substanzen sowie aufgrund der Robustheit der Einrichtung wegen des an sich sehr widerstandsfähigen Materials Diamant für eine Vielzahl von Anwendungen. Insbesondere kann die erfindungsgemäße Einrichtung auch unter sehr schwierigen Bedingungen, beispielsweise bei hohen Temperaturen oder in reaktiven Milieus eingesetzt werden. Die Anregung der sensitiven Komponente durch elektromagnetische Strahlung und das optische Auslesen des Sensors ermöglicht darüber hinaus einen Einsatz in schwer zugänglichen Bereichen, da durch diese Art der Anregung und des Auslesens keine direkte Ankopplung an elektronische Elemente erforderlich ist.

Die Analyse der ausgeatmeten Luft (Atemgas) stellt eine nicht-invasive Methode im Rahmen der medizinischen Diagnostik dar. Dem liegt zugrunde, dass die Lunge den Stoffaustausch zwischen Blutkreislauf und Atemluft vermittelt, so dass Bestandteile des Atemgases biochemische Vorgänge im Körper widerspiegeln. So können beispielsweise bestimmte Biomarker, die im Rahmen einer Früherkennung oder einer Verlaufs- und Therapiekontrolle von Krankheiten eine Rolle spielen, untersucht werden. Eine Analyse von Bestandteilen des Atemgases erfolgt herkömmlicherweise oftmals mit massenspektrometrischen Methoden, wobei in der Regel hierfür ein hoher apparativer Aufwand erforderlich ist. Die erfindungsgemäße Analyseeinrichtung in ihrer Verwendung für ein Atemgasanalysegerät erlaubt hingegen eine miniaturisierte Analytik, die idealerweise sogar tragbar ist. Hiermit lässt sich ein regelmäßiges Monitoring der für den jeweiligen Krankheitsverlauf relevanten Bestandteile des Atemgases für den Patienten realisieren.

In einer bevorzugten Ausgestaltung des Atemgasanalysegeräts umfasst das Gerät Mittel zur Kondensation der Ausatemluft, insbesondere eine Kühlfalle. Mit dieser Massnahme wird das Atemgas kondensiert, so dass die nachfolgende Analytik auf der Basis der Messung von magnetischen Kernspinresonanzen in der Flüssigphase erfolgen kann. Vorzugsweise kann sich die Kühlfalle im Bereich der sensitiven Komponente des Magnetfeldsensors befinden. Beispielsweise kann ein übliches Kühlelement unterhalb der sensitiven Komponente angeordnet werden, sodass sich das Kondensat zweckmäßigerweise direkt auf den Diamantstrukturen mit den NV-Zentren abscheidet. Das Kühlelement kann mit einem Heizelement kombiniert werden. Durch ein Heizelement kann wiederum eine Entfernung der adsorbierten Flüssigkeit nach der Messung und ein Reset des Sensors erreicht werden. Zu diesem Zweck kann beispielsweise ein Peltier-Element mit integriertem Heizer vorgesehen sein.

Beispielsweise kann ein solches Atemgasanalysegerät für den Nachweis von Wasserstoffperoxid genutzt werden, welches als Entzündungsindikator im Atemgas betrachtet wird. Beispielsweise wurden signifikante Erhöhungen der Wasserstoffperoxidkonzentration im Atemgas bei Patienten mit Asthma bronchiale oder chronisch obstruktiven Lungenerkrankungen nachgewiesen. Es sind bereits verschiedene Verfahren bekannt, um Wasserstoffperoxid im Atemgas nachzuweisen, wobei in der Regel eine Kondensation des Atemgases und eine anschließende Flüssigphasenanalytik vorgenommen wird. Beispielsweise sind amperometrische Messungen oder optische Verfahren bekannt. Entsprechende Messvorrichtungen sind jedoch für den Laborbetrieb ausgelegt und können nicht selbst vom Patienten vorgenommen werden. Durch den Einsatz der erfindungsgemäßen Analyseeinrichtung in einem Atemgasanalysegerät kann hingegen eine miniaturisierte Form eines solchen Geräts bereitgestellt werden, das beispielsweise auch dem Patienten selbst eine entsprechende Überwachung im täglichen Leben erlaubt. Darüber hinaus hat das erfindungsgemäße Atemgasanalysegerät den Vorteil, dass keine Reaktionsreagenzien oder funktionalisierte Oberflächen vorgehalten werden müssen, die sich nach der Reaktion verbrauchen würden und zu ersetzen wären. Das erfindungsgemäße Atemgasanalysegerät ist nicht auf einen bestimmten Analyten beschränkt. Vielmehr können durch Anpassung der Auswertung auch andere Analyten, wie beispielsweise Schwefelwasserstoff, das als Indikator von Halitosis gilt, untersucht werden. Allgemein kann das Atemgasanalysegerät auch für die Analyse von anderen diagnostisch relevanten Substanzen oder Molekülen eingesetzt werden. Geeignet hierfür sind im Prinzip vor allem Moleküle, die Wasserstoffatome (H) und/oder Kohlenstoff-Atome (C) enthalten, da ¹H und ¹³C Isotope sind, die sehr gut mit der erfindungsgemäß eingesetzten magnetischen Kernspinresonanzmessung nachweisbar sind.

Die Erfindung umfasst weiterhin ein Verfahren zur Analyse von Substanzen in einer Atemgasprobe auf der Basis einer Messung von magnetischen Kernspin-Resonanzen mit einem erfindungsgemäßen Atemgasanalysegerät. Das Verfahren ist dadurch gekennzeichnet, dass ein Magnetfeld erzeugt wird, welches Magnetresonanzen in der Probe bzw. in bestimmten Substanzen in der Probe erzeugt. Zur Detektion der erzeugten Magnetresonanzen werden die Diamantstrukturen mit Stickstoff-Vakanz-Zentren verwendet. Vorteilhafterweise werden bei Einkopplung von elektromagnetischer Strahlung im optischen Bereich und bei Einkopplung von elektromagnetischer Strahlung im Mikrowellenbereich eine von den Diamantstrukturen emittierte Fluoreszenzstrahlung als Maß für die induzierten Magnetresonanzen ausgewertet. Vorzugsweise wird hierbei die elektromagnetische Strahlung im Mikrowellenbereich mit variierender Frequenz eingekoppelt. In dem daraus resultierenden Fluoreszenzspektrum, das von den NV-Zentren der Diamantstrukturen emittiert wird, resultieren dabei charakteristische Minima bzw. Einbrüche der Fluoreszenz. Diese Minima werden in Bezug zu der Frequenz der Mikrowellenstrahlung ausgewertet. Je nach wirkendem Magnetfeld wird eine bestimmte Verschiebung der Fluoreszenzminima induziert. Die Position der Fluoreszenzminima innerhalb des Fluoreszenzspektrums kann damit als Maß für das wirkende Magnetfeld ausgewertet werden. Bezüglich weiterer Merkmale des erfindungsgemäßen Verfahrens wird auch auf die obige Beschreibung verwiesen.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

In den Figuren zeigen
- Fig. 1: schematische Darstellung eines Stickstoff-Vakanz-Zentrums in Diamant;
- Fig. 2: schematische Schnittdarstellung einer Ausführungsform einer erfindungsgemäßen Analyseeinrichtung als monolithisch integriertes NMR-Sensorelement;
- Fig. 3: schematische Schnittdarstellung einer weiteren Ausführungsform einer erfindungsgemäßen Analyseeinrichtung als monolithisch integriertes NMR-Sensorelement für eine Verwendung in einem erfindungsgemäßen Atemgasanalysegerät und
- Fig. 4: schematische Darstellung eines erfindungsgemäßen Atemgasanalysegerät.

### Beschreibung von Ausführungsbeispielen

Kern der Erfindung ist die Ausnutzung von Stickstoff-Vakanz-Zentren in Diamant zur Messung von magnetischen Kernspinresonanzen, wodurch sehr empfindliche Messeinrichtungen bereitgestellt werden können, die insbesondere auch für miniaturisierte Anwendungen geeignet sind.

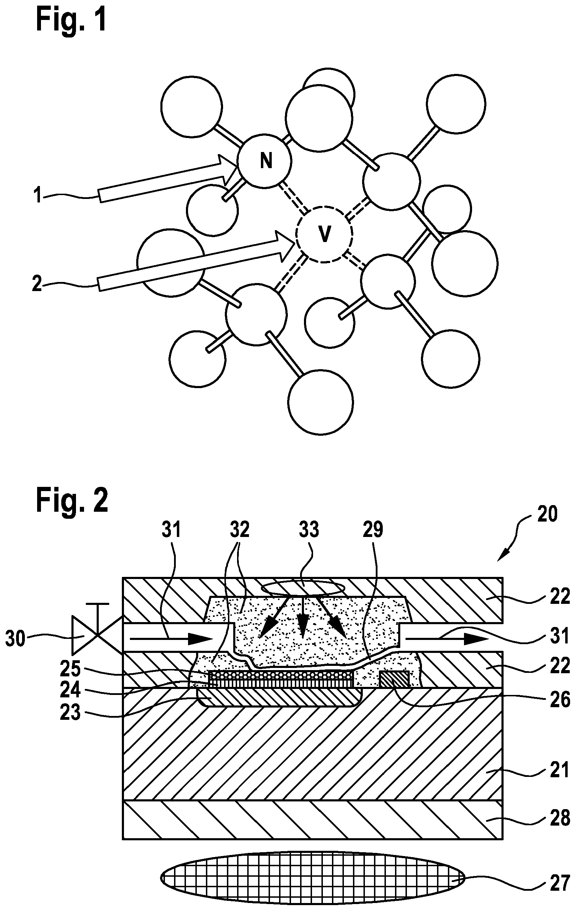

**Fig. 1** illustriert das an sich bekannte Stickstoff-Vakanz-Zentrum (NV-Zentrum) in Diamant. Dargestellt ist das Kohlenstoff-Atomgitter, das die Diamantstruktur bildet. Eines der Kohlenstoff-Atome ist durch ein Stickstoff-Atom N (Pfeil 1) ersetzt. Ein direkt benachbartes Kohlenstoff-Atom fehlt im Diamantgitter. Dies ist in dieser Darstellung mit V (*Vacancy*) (Pfeil 2) bezeichnet. Ein solches NV-Zentrum in Diamant besitzt bei Raumtemperatur ein bestimmtes Energiespektrum. Im Normalzustand, d.h. bei Anregung mit Licht im optischen Bereich und ohne eine weitere Bestrahlung im Mikrowellenbereich und ohne Anlegen eines magnetischen Feldes, zeigt das NV-Zentrum bei optischer Anregung eine Fluoreszenz im roten Wellenlängenbereich. Wird neben der optischen Anregung zusätzlich noch eine Mikrowellenbestrahlung eingekoppelt, kommt es bei einer bestimmten Frequenz, insbesondere bei 2,88 GHz, zu einem Einbruch der Fluoreszenz, also zu einem Fluoreszenz-Minimum, das messbar ist. Dieses Phänomen lässt sich damit begründen, dass die Elektronen des NV-Zentrums in diesem Fall von dem Niveau mₛ = ± 1 des ³A-Zustandes auf das Niveau mₛ = ± 1 des ³E-Zustandes gehoben werden und von dort nichtstrahlend rekombinieren. Beim Anlegen eines externen Magnetfeldes kommt es zu einer Aufspaltung des Niveaus mₛ = ± 1 (*Zeeman-Splitting*) und es zeigen sich bei Auftragen der Fluoreszenz über die Frequenz der Mikrowellenanregung zwei Minima im Fluoreszenzspektrum, deren Frequenzabstand proportional zur magnetischen Feldstärke ist (Balasubramanian et al., Nature, Vol. 455, Seite 648 (2008)). Die Magnetfeldsensitivität wird dabei durch die minimal-auflösbare Frequenzverschiebung vorgegeben und kann bis zu 100 pT/√Hz erreichen. Bei einer Übereinstimmung der Mikrowellenfrequenz mit dem Energieabstand zwischen dem Niveau mₛ = 0 und mₛ = ± 1 kommt es also zu einem Einbruch der Fluoreszenz. Bei einem externen Magnetfeld spaltet das Niveau mₛ = ± 1 auf und es sind zwei definierte Mikrowellenfrequenzen zu beobachten, bei denen die Fluoreszenz abnimmt (Minima). Der Frequenzabstand bei diesen definierten Mikrowellenfrequenzen ist dabei proportional zum Magnetfeld, so dass durch Auswertung der Fluoreszenzminima auf die Magnetfeldstärke rückgeschlossen werden kann.

Die an sich bekannte Kernspinresonanzspektroskopie beruht darauf, dass viele Atome oder Isotope ein magnetisches Moment in ihrem Kernspin besitzen. Zu diesen Isotopen gehören die natürlich vorkommenden ¹H-Isotope beispielsweise in Wasserstoffperoxid (H₂O₂) sowie das in allen organischen Verbindungen enthaltene ¹³C-Isotop. Diese magnetischen Momente sind ohne äußere Anregung statisch ausgerichtet. Durch Anlegen eines externen Magnetfeldes beginnen diese Spins zu präzidieren und es tritt ein magnetisches Wechselfeld mit charakteristischer Frequenz auf. Die Frequenz kann der jeweiligen Atomspezies und dem Bindungszustand zugeordnet werden. Das der Erfindung zu Grunde liegende Messprinzip detektiert die charakteristischen Frequenzen mit Hilfe eines Magnetfeldsensors, der auf Diamantstrukturen mit NV-Zentren basiert. Die Kernspinresonanzen sind in den Fluoreszenzspektren der NV-Zentren als Rauschen sichtbar. Durch gezielte Anrege-Abfragesequenzen, sogenannte *XY8N-Decoupling Sequences,* können die Kernspinresonanzen aus dem Rauschen herausgefiltert werden. Die Analyse dieser Rauschspektren erlaubt dann wie in der klassischen Kernspinresonanzspektroskopie eine chemische Analyse der Probenbestandteile. Es konnte bereits von Staudacher et al. (Science, Vol. 339, Seite 561-563 (2013)) gezeigt werden, dass beispielsweise sowohl eine Unterscheidung zwischen ¹³C und ¹H als auch eine Unterscheidung verschiedener Substanzen mit dem erfindungsgemäßen Messprinzip möglich ist.

**Fig. 2** zeigt eine mögliche Ausführungsform eines erfindungsgemäßen monolithisch integrierten NMR-Sensorelements als erfindungsgemäße Analyseeinrichtung 20. Auf einem Trägersubstrat 21, beispielsweise einem Silizium-Substrat, ist eine LED-Struktur 22 mit einer integrierten Lichtquelle 33 gebondet. Alternativ kann statt einer LED-Struktur beispielsweise ein VCSEL-Laserchip vorgesehen sein. Die Lichtquelle 33 sollte vorzugsweise eine Wellenlänge im grünen Bereich des sichtbaren Lichtspektrums abstrahlen, insbesondere im Wellenlängenbereich zwischen etwa 530 nm bis etwa 570 nm, da in diesem Bereich die Photonenabsorption der NV-Zentren maximal ist. Das Trägersubstrat 21 umfasst weiterhin eine Photodiode 23, beispielsweise eine p-n-Photodiode, oder eine Anordnung von mehreren Photodioden. Oberhalb der Photodiode befindet sich eine optische Filterschicht 24. Oberhalb der Filterschicht 24 ist eine mit NV-Zentren versetzte Diamantschicht 25 als sensitive Komponente der Analyseeinrichtung 20 angeordnet. In räumlicher Nähe zur Diamantschicht 25 ist eine RF-Antenne als Mittel 26 zur Einkopplung der Mikrowellenstrahlung vorgesehen. Im Zusammenspiel mit einem *Voltage Controlled Oscillator* (VCO) kann über die Mikrowellenantenne 26 die erforderliche elektromagnetische Anregungsstrahlung im Mikrowellenbereich eingekoppelt werden. Bei der Antenne 26 kann es sich beispielsweise um eine RF-Streifenantenne handeln, die am Rand der Photodioden-Anordnung 23 platziert ist. Bei gleichzeitiger elektromagnetischer Anregungsstrahlung im optischen Bereich mittels der Lichtquelle 33 wird durch die charakteristischen Kernspinresonanzen in den NV-Zentren ein charakteristisches Fluoreszenzspektrum emittiert, das über die Photodiode 23 detektierbar ist. Hierbei filtert die optische Filterschicht 24 das Anregungslicht aus dem Fluoreszenzspektrum heraus. Als Messsignal wird dann die Veränderung der Fluoreszenzintensität bei einer Anregung mit der Anrege-Abfragesequenz (*XY8N-Decoupling Sequence*) ausgewertet.

Da die minimal auflösbare Frequenzverschiebung mit der Lebensdauer der angeregten Spin-Zustände in den NV-Zentren zusammenhängt, kann es von Vorteil sein, zur Messung der Kernspinresonanzen den Effekt der Kopplung von NV-Elektronenspins an dem N-Kernspin zu nutzen, und die Frequenzinformation an den ml = 1 N-Kernspin-Zustand zu übertragen, welcher eine Lebensdauer von Tagen besitzt, wohingegen die Lebensdauer der angeregten Spin-Zustände in den NV-Zentren im Bereich von Millisekunden liegt (Laraoui et al., Nature Comm., DOI: 10.1038/ncomms2685). Durch diese Maßnahme lassen sich prinzipiell Frequenzverschiebungen in den zu messenden Kernspinresonanzen von wenigen Hz auflösen.

Im unteren Teil der dargestellten schematischen Schnittansicht ist ein Referenzmagnet 27 gezeigt, der beispielsweise ein Magnetfeld mit einer Stärke von 100 mT bereitstellen kann. Der Referenzmagnet 27 wird insbesondere von einer Spule gebildet, die sich in räumlicher Nähe zu den übrigen Komponenten der Analyseeinrichtung befindet. Mittels des Magneten 27 wird das externe Magnetfeld erzeugt, das zur Anregung der charakteristischen Spin-Präzession erforderlich ist. Im Vergleich mit üblichen NMR-Geräten kann der Referenzmagnet 27 wesentlich kleiner ausgelegt werden, da im Gegensatz zur klassischen NMR kein Kollektiv an gleichgerichteten Kernspins in einem relativ großen Probenvolumen synchronisiert werden muss, sondern individuelle Kernspins in einem kleinen Probenvolumen ausgelesen werden.

Durch eine Strukturierung der Diamantschicht 25 beispielsweise in Form von Röhren oder Nadeln kann die Oberfläche vergrößert werden, wodurch die Sensitivität erhöht werden kann. Durch Kapillarstrukturen in der Diamantschicht 25 kann die Probe für einen längeren Zeitraum festgehalten werden, wodurch die Messzeit verlängert und damit die Messbarkeit verbessert werden kann.

Die Analyseeinrichtung 20 umfasst weiterhin ein Element 28 zum Heizen und/oder Kühlen. Durch ein Beheizen der Einrichtung nach der erfolgten Messung können beispielsweise Probenrückstände auf der Diamantschicht 25 entfernt werden. Wenn die Diamantschicht 25 beispielsweise Kapillaren enthält, um eine Probe länger zurückzuhalten, kann durch ein Erhitzen der Einrichtung mittels des Heizelements 28 die Probenflüssigkeit nach der Messung verdampft werden. Dies kann für eine Regenerierung des Sensors genutzt werden. Eine Kühlung mittels des Elements 28 kann eingesetzt werden, um nach einem Beheizen wieder definierte Messbedingungen einzustellen. Weiterhin kann eine Kühlung im Sinne einer Kühlfalle eingesetzt werden, um eine gegebenenfalls gasförmige Probe zu kondensieren.

Vorteilhafterweise ist eine Mehrzahl von individuell auszuwertenden NV-Zentren oder NV-Sensoren in der Diamantschicht 25 enthalten. Entsprechend kann auch die Photodiode 23 als Anordnung von mehreren Photodioden ausgestaltet sein. Durch diese Maßnahme kann die Konzentrationssensitivität für eine bestimmte Molekülspezies weiter erhöht werden.

Die zu analysierende Probe wird über mikrofluidische Kanäle 29 über die Diamantschicht 25 geführt. Hierbei kann es vorgesehen sein, dass während der Messung der Probenstrom unterbrochen wird, dies wird durch Schaltung eines entsprechenden Einlassventils 30 realisiert. Während der Messung wird das Einlassventil 30 also geschlossen, um die zu analysierende Probe im Bereich der sensitiven Komponente 25, also der Diamantschicht, zu halten. Nach der Messung wird das Ventil 30 geöffnet, so dass die zu messende Probe in den Kanälen 29 über eine Druckdifferenz zwischen dem Einlass des Sensors im Bereich des Einlassventils 30 und zwischen dem entgegengesetzt liegenden Auslass, hier angedeutet durch einen Pfeil 31, ausgetauscht werden kann. Das Material 32, das von den Kanälen 29 durchzogen ist, ist zweckmäßigerweise optisch transparent, so dass die optische Anregungsstrahlung aus der Lichtquelle 33 die NV-Zentren in der Diamantschicht 25 ohne wesentliche Verluste erreichen kann.

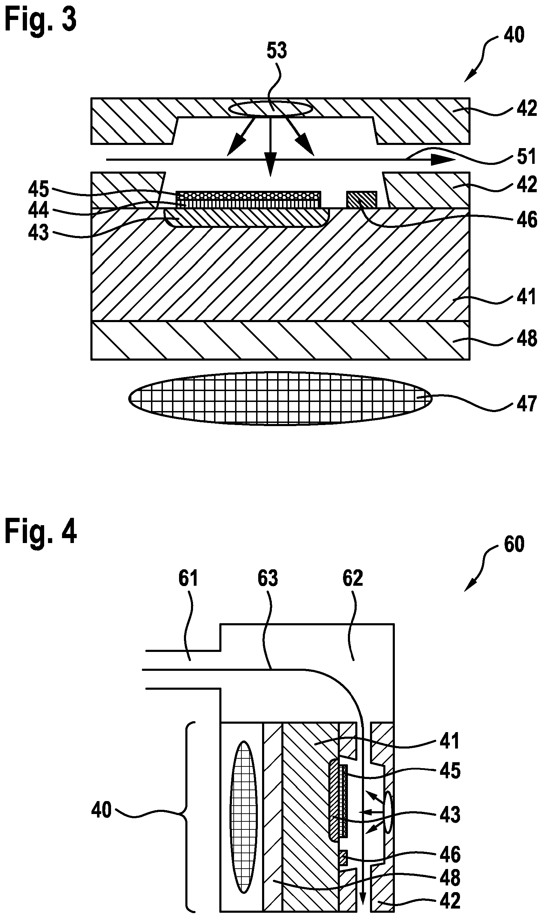

**Fig. 3** illustriert ein weiteres Beispiel für eine erfindungsgemäße Analyseeinrichtung 40, die in ein Atemgasanalysegerät integriert werden kann. Vergleichbar mit der in Fig. 2 illustrierten Ausgestaltung umfasst die Analyseeinrichtung 40 ein Trägersubstrat 41, in das ein Photodioden-Array 43 integriert ist. Oberhalb des Bereichs mit dem Photodioden-Array 43 befindet sich eine optische Filterschicht 44. Auf der optischen Filterschicht 44 ist eine Diamantschicht 45 mit integrierten NV-Zentren (sensitive Komponente) angeordnet. In räumlicher Nähe zu der Diamantschicht 45 befindet sich eine Mikrowellen-Antenne 46 zur Einkopplung der elektromagnetischen Anregungsstrahlung im Mikrowellenbereich. Auf dem Trägersubstrat 41 ist eine LED-Struktur 42 (oder eine VCSEL-Struktur) mit einer Lichtquelle 53 zur Abstrahlung von elektromagnetischer Strahlung im optischen Bereich angeordnet. Die Struktur 42 ist so strukturiert, dass sie von der zu analysierenden Probe durchflossen werden kann, hier angedeutet durch den Pfeil 51. Unterhalb des Trägersubstrats 41 befindet sich ein Element 48 zum Kühlen und Heizen der Analyseeinrichtung 40. Hierbei kann es sich beispielsweise um ein beheizbares Peltier-Element handeln. In räumlicher Nähe zu diesen Strukturen befindet sich ein Magnet 47, der zur Erzeugung des Referenzmagnetfeldes vorgesehen ist, um die Spin-Präzession und die Magnetresonanzen in den NV-Zentren der Diamantschicht 45 zu induzieren. Für die erfindungsgemäße Analyseeinrichtung ist beispielsweise ein Magnetfeld von B = 100 mT ausreichend, da das erfindungsgemäß eingesetzte Messprinzip über die NV-Zentren in Diamant extrem empfindlich ist. Die gesamten Abmessungen einer solchen monolithisch integrierbaren NMR-Sensorstruktur können beispielsweise etwa 5 cm x 5 cm betragen. Damit ist die erfindungsgemäße Analyseeinrichtung für miniaturisierte Anwendungen sehr geeignet. Beispielsweise kann eine solche Analyseeinrichtung in ein Atemgasanalysegerät eingebaut werden, das handlich ist und von einem Patienten im täglichen Bedarf eingesetzt werden kann.

Insbesondere bei Einrichtungen, die für eine Analyse von gasförmigen Proben, wie z. B. Atemgas, eingesetzt werden, ist zweckmäßigerweise eine Kondensation der gasförmigen Probe vorgesehen. Bei der Einrichtung 40 kann hierfür das Element 48 zur Realisierung einer Kühlfalle eingesetzt werden. Wenn die gasförmige Probe durch die Kanäle der Struktur 42 geführt wird (Pfeil 51), wird der Träger 41 zusammen mit der darauf angeordneten sensitiven Diamantschicht 45 gekühlt, sodass die Probe direkt auf der Diamantschicht 45 kondensiert. Die Analyse kann anschließend in einer Flüssigphase der Probe erfolgen. Nach erfolgter Messung kann insbesondere der Bereich der Diamantschicht 45 mittels des kombinierten Heiz- und Kühlelements 48 aufgeheizt werden, sodass das Kondensat durch Verdampfen wieder entfernt wird, und die Analyseeinrichtung 40 für eine erneute Messung vorbereitet werden kann.

**Fig. 4** illustriert die Integration der Analyseeinrichtung 40 aus Fig. 3 in ein Atemgasanalysegerät 60. Das Atemgasanalysegerät weist ein Mundstück 61 auf, über das die Ausatemluft von einem Patienten oder allgemein von einem Anwender in das Gerät 60 eingeblasen wird. Das Mundstück kann aus hygienischen Gründen mit einem Keimfilter ausgestattet sein. Das Atemgasanalysegerät 60 umfasst weiterhin eine Analysekammer 62, die die anhand von Fig. 3 bereits erläuterte Analyseeinrichtung 40 (Messeinrichtung) enthält. Über eine entsprechende Luftführung 63 innerhalb des Geräts gelangt die Luft in die Kanalstrukturen der Analyseeinrichtung 40, so dass die Ausatemluft (Atemgas) über die Diamantschicht 45 der Analyseeinrichtung 40 geführt wird. Durch das kombinierte Heiz- und Kühlelement 48 wird hierbei eine Kühlfalle bereitgestellt, so dass das Atemgas, wenn es in den Bereich der Diamantschicht 45 gelangt, kondensiert und in der Flüssigphase gemäß dem erfindungsgemäßen Prinzip analysiert werden kann.

## Patentansprüche

1. Atemgasanalysegerät (40, 60) zur Analyse von Substanzen in einer Atemgasprobe auf der Basis einer Messung von magnetischen Kernspinresonanzen, umfassend ein Mundstück (61) zum Einblasen der Atemgasprobe und eine über eine Luftführung (63) verbundene Analyseeinrichtung (20; 40), wobei die Analyseeinrichtung Mittel (27; 47) zur Erzeugung eines Magnetfeldes umfasst, wobei zur Detektion von Magnetresonanzen, die durch die Erzeugung des Magnetfeldes in der Probe induziert werden, wenigstens ein Magnetfeldsensor vorgesehen ist, der wenigstens eine sensitive Komponente (25; 45) mit Diamantstrukturen umfasst, wobei die Diamantstrukturen Stickstoff-Vakanz-Zentren aufweisen, wobei der Magnetfeldsensor wenigstens ein Mittel (33; 53) zur Einkopplung von elektromagnetischer Anregungsstrahlung im optischen Bereich, insbesondere in einem Wellenlängenbereich zwischen etwa 530 nm und etwa 570 nm, und wenigstens ein Mittel (26; 46) zur Einkopplung von elektromagnetischer Strahlung im Mikrowellenbereich, insbesondere in einem Frequenzbereich zwischen etwa 2000 MHz und etwa 4000 MHz, und wenigstens ein Mittel (23; 43) zur Detektion von emittierter Fluoreszenzstrahlung aufweist.

2. Atemgasanalysegerät (40, 60) nach Anspruch 1, **dadurch gekennzeichnet, dass** der Magnetfeldsensor wenigstens ein Mittel (24; 44) zur Filterung von elektromagnetischer Strahlung aufweist, wobei vorzugsweise das Mittel zur Filterung von elektromagnetischer Strahlung wenigstens eine optische Filterschicht zur Filterung der Anregungsstrahlung aus der emittierten Fluoreszenzstrahlung ist.

3. Atemgasanalysegerät (40, 60) nach einem der vorhergehenden Ansprüche, **dadurch gekennzeichnet, dass** der Magnetfeldsensor eine Anordnung von mehreren individuell auszuwertenden sensitiven Komponenten (24; 44) umfasst.

4. Atemgasanalysegerät (40, 60) nach einem der vorhergehenden Ansprüche, **dadurch gekennzeichnet, dass** die sensitive Komponente (25; 45) Strukturen zur Oberflächenvergrößerung aufweist, insbesondere nadelartige oder röhrenartige Strukturen.

5. Atemgasanalysegerät (40, 60) nach einem der vorhergehenden Ansprüche, **dadurch gekennzeichnet, dass** die sensitive Komponente (25; 45) Kapillarstrukturen aufweist.

6. Atemgasanalysegerät (40, 60) nach einem der vorhergehenden Ansprüche, **dadurch gekennzeichnet, dass** der sensitiven Komponente (25; 45) wenigstens ein Element (28; 48) zum Heizen und/oder Kühlen, insbesondere ein heizbares Peltier-Element, zugeordnet ist.

7. Atemgasanalysegerät (40, 60) nach einem der vorhergehenden Ansprüche, **dadurch gekennzeichnet, dass** das Atemgasanalysegerät (40, 60) Mittel zur Ionisation von Substanzen in der Probe umfasst.

8. Atemgasanalysegerät (40, 60) nach einem der vorhergehenden Ansprüche, **dadurch gekennzeichnet, dass** das Atemgasanalysegerät (60) Mittel zur Erzeugung eines positiven Potenzials an der sensitiven Komponente (25; 45) umfasst.

9. Atemgasanalysegerät (40, 60) nach einem der vorhergehenden Ansprüche, **dadurch gekennzeichnet, dass** das Atemgasanalysegerät Mittel (48) zur Kondensation der Ausatemluft, insbesondere eine Kühlfalle, umfasst, wobei vorzugsweise die Mittel zur Kondensation der Atemluft Mittel zur Kühlung der sensitiven Komponente (45) sind.

10. Atemgasanalysegerät (40, 60) nach einem der vorhergehenden Ansprüche, **dadurch gekennzeichnet, dass** das Atemgasanalysegerät zur Messung von Wasserstoffperoxid und/oder Schwefelwasserstoff eingerichtet ist.

11. Verfahren zur Analyse von Substanzen in einer Atemgasprobe auf der Basis einer Messung von magnetischen Kernspinresonanzen mit einem Atemgasanalysegerät (40, 60) nach einem der vorhergehenden Ansprüche, **dadurch gekennzeichnet, dass** ein Magnetfeld erzeugt wird, welches Magnetresonanzen in der Atemgasprobe induziert, und wobei zur Detektion der induzierten Magnetresonanzen die Diamantstrukturen des Atemgasanalysegeräts (40, 60) verwendet werden.

12. Verfahren nach Anspruch 11, **dadurch gekennzeichnet, dass** bei Einkopplung von elektromagnetischer Strahlung im optischen Bereich und bei Einkopplung von elektromagnetischer Strahlung im Mikrowellenbereich eine von den Diamantstrukturen emittierte Fluoreszenzstrahlung als Maß für die induzierten Magnetresonanzen ausgewertet wird, wobei vorzugsweise die elektromagnetische Strahlung im Mikrowellenbereich mit variierender Frequenz eingekoppelt wird und daraus resultierende Minima in der von den Diamantstrukturen emittierten Fluoreszenzstrahlung in Bezug zu der Frequenz der elektromagnetischen Strahlung im Mikrowellenbereich ausgewertet werden.

## Claims

1. Respiratory gas analyser (40, 60) for analysis of substances in a respiratory gas sample on the basis of a measurement of magnetic nuclear spin resonances, comprising a mouthpiece (61) for blowing in the respiratory gas sample and an analysis device (20; 40) connected via an air feed (63), wherein the analysis device comprises means (27; 47) of generating a magnetic field, wherein the detection of magnetic resonances that are induced by the generation of the magnetic field in the sample is accomplished by provision of at least one magnetic field sensor comprising at least one sensitive component (25; 45) having diamond structures, wherein the diamond structures have nitrogen vacancy sites, wherein the magnetic field sensor has at least one means (33; 53) of coupling in electromagnetic excitation radiation in the optical region, especially in a wavelength range between about 530 nm and about 570 nm, and at least one means (26; 46) of coupling in electromagnetic radiation in the microwave region, especially in a frequency range between about 2000 MHz and about 4000 MHz, and at least one means (23; 43) of detecting emitted fluorescence radiation.

2. Respiratory gas analyser (40, 60) according to Claim 1, **characterized in that** the magnetic field sensor has at least one means (24; 44) of filtering electromagnetic radiation, wherein the means of filtering electromagnetic radiation is preferably at least one optical filter layer for filtering of the excitation radiation out of the emitted fluorescence radiation.

3. Respiratory gas analyser (40, 60) according to either of the preceding claims, **characterized in that** the magnetic field sensor comprises an arrangement of multiple, individually evaluable sensitive components (24; 44).

4. Respiratory gas analyser (40, 60) according to any of the preceding claims, **characterized in that** the sensitive component (25; 45) has structures for increasing surface area, especially needle-like or tube-like structures.

5. Respiratory gas analyser (40, 60) according to any of the preceding claims, **characterized in that** the sensitive component (25; 45) has capillary structures.

6. Respiratory gas analyser (40, 60) according to any of the preceding claims, **characterized in that** the sensitive component (25; 45) has at least one assigned element (28; 48) for heating and/or cooling, especially a heatable Peltier element.

7. Respiratory gas analyser (40, 60) according to any of the preceding claims, **characterized in that** the respiratory gas analyser (40, 60) has means of ionizing substances in the sample.

8. Respiratory gas analyser (40, 60) according to any of the preceding claims, **characterized in that** the respiratory gas analyser (60) comprises means of generating a positive potential on the sensitive component (25; 45).

9. Respiratory gas analyser (40, 60) according to any of the preceding claims, **characterized in that** the respiratory gas analyser comprises means (48) of condensing the exhaled air, especially a cold trap, wherein the means of condensing the exhaled air are preferably means of cooling the sensitive component (45) .

10. Respiratory gas analyser (40, 60) according to any of the preceding claims, **characterized in that** the respiratory gas analyser is set up to measure hydrogen peroxide and/or hydrogen sulfide.

11. Method of analysing substances in a respiratory gas sample on the basis of a measurement of magnetic nuclear spin resonances with a respiratory gas analyser (40, 60) according to any of the preceding claims, **characterized in that** a magnetic field that induces magnetic resonances in the respiratory gas sample is generated, and wherein the magnetic resonances are induced using the diamond structures of the respiratory gas analyser (40, 60).

12. Method according to Claim 11, **characterized in that**, when coupling in electromagnetic radiation in the optical region and when coupling in electromagnetic radiation in the microwave region, a fluorescence radiation emitted by the diamond structures is evaluated as a measure of the magnetic resonances induced, preferably by coupling in the electromagnetic radiation in the microwave region with varying frequency and evaluating minima resulting therefrom in the fluorescence radiation emitted by the diamond structures in relation to the frequency of the electromagnetic radiation in the microwave region.

## Revendications

1. Appareil d'analyse de gaz respiratoire (40, 60) destiné à analyser des substances dans un échantillon de gaz respiratoire sur la base d'une mesure de résonances magnétiques nucléaires, comprenant un embout buccal (61) servant à insuffler l'échantillon de gaz respiratoire et un dispositif d'analyse (20 ; 40) relié par le biais d'un conduit d'air (63), le dispositif d'analyse comportant des moyens (27 ; 47) destinés à générer un champ magnétique, au moins un capteur de champ magnétique étant présent pour la détection des résonances magnétiques qui sont induites par la génération du champ magnétique dans l'échantillon, lequel comporte au moins un composant sensible (25 ; 45) ayant des structures en diamant, les structures en diamant possédant des centres de vacance d'azote,
le capteur de champ magnétique possédant au moins un moyen (33 ; 53) destiné à injecter par couplage un rayonnement d'excitation électromagnétique dans la plage optique, notamment dans une plage de longueurs d'onde entre environ 530 nm et environ 570 nm, et au moins un moyen (26 ; 46) destiné à injecter par couplage un rayonnement électromagnétique dans la plage des hyperfréquences, notamment dans une plage de fréquences entre environ 2000 MHz et environ 4000 MHz, et au moins un moyen (23 ; 43) destiné à détecter un rayonnement de fluorescence émis.

2. Appareil d'analyse de gaz respiratoire (40, 60) selon la revendication 1, **caractérisé en ce que** le capteur de champ magnétique possède au moins un moyen (24 ; 44) destiné à filtrer le rayonnement électromagnétique, le moyen destiné à filtrer le rayonnement électromagnétique étant de préférence au moins une couche de filtrage optique destinée à filtrer le rayonnement d'excitation hors du rayonnement de fluorescence émis.

3. Appareil d'analyse de gaz respiratoire (40, 60) selon l'une des revendications précédentes, **caractérisé en ce que** le capteur de champ magnétique comporte un arrangement composé de plusieurs composants sensibles (24 ; 44) à interpréter individuellement.

4. Appareil d'analyse de gaz respiratoire (40, 60) selon l'une des revendications précédentes, **caractérisé en ce que** le composant sensible (25 ; 45) possède des structures servant au grossissement de la surface, notamment des structures de type aiguilles ou de type tubes.

5. Appareil d'analyse de gaz respiratoire (40, 60) selon l'une des revendications précédentes, **caractérisé en ce que** le composant sensible (25 ; 45) possède des structures capillaires.

6. Appareil d'analyse de gaz respiratoire (40, 60) selon l'une des revendications précédentes, **caractérisé en ce qu'**au moins un élément (28 ; 48) servant au chauffage et/ou au refroidissement, notamment un élément de Peltier pouvant être chauffé, est associé au composant sensible (25 ; 45).

7. Appareil d'analyse de gaz respiratoire (40, 60) selon l'une des revendications précédentes, **caractérisé en ce que** l'appareil d'analyse de gaz respiratoire (40, 60) comporte des moyens destinés à l'ionisation de substances dans l'échantillon.

8. Appareil d'analyse de gaz respiratoire (40, 60) selon l'une des revendications précédentes, **caractérisé en ce que** l'appareil d'analyse de gaz respiratoire (60) comporte des moyens destinés à générer un potentiel positif au niveau du composant sensible (25 ; 45).

9. Appareil d'analyse de gaz respiratoire (40, 60) selon l'une des revendications précédentes, **caractérisé en ce que** l'appareil d'analyse de gaz respiratoire comporte des moyens (48) destinés à la condensation de l'air respiratoire expiré, notamment un piège cryogénique, les moyens destinés à la condensation de l'air respiratoire étant de préférence des moyens destinés à refroidir le composant sensible (45).

10. Appareil d'analyse de gaz respiratoire (40, 60) selon l'une des revendications précédentes, **caractérisé en ce que** l'appareil d'analyse de gaz respiratoire est conçu pour mesurer le peroxyde d'hydrogène et/ou l'hydrogène sulfuré.

11. Procédé pour analyser des substances dans un échantillon de gaz respiratoire sur la base d'une mesure de résonances magnétiques nucléaires avec un appareil d'analyse de gaz respiratoire (40, 60) selon l'une des revendications précédentes, **caractérisé en ce qu'**un champ magnétique est généré, lequel induit des résonances magnétiques dans l'échantillon de gaz respiratoire, et les structures en diamant de l'appareil d'analyse de gaz respiratoire (40, 60) étant utilisées pour la détection des résonances magnétiques induites.

12. Procédé selon la revendication 11, **caractérisé en ce que** lors de l'injection par couplage d'un rayonnement électromagnétique dans la plage optique et lors de l'injection par couplage d'un rayonnement électromagnétique dans la plage des hyperfréquences, un rayonnement de fluorescence émis par les structures en diamant est interprété comme une mesure des résonances magnétiques induites, le rayonnement électromagnétique dans la plage des hyperfréquences étant de préférence injecté par couplage avec une fréquence variable et les minima qui en résultent dans le rayonnement de fluorescence émis par les structures en diamant étant interprétés en référence à la fréquence du rayonnement électromagnétique dans la plage des hyperfréquences.
